(19) Europäisches Patentamt
European Patent Office
Office européen des brevets

(11) **EP 2 465 418 B1**

(12) **FASCICULE DE BREVET EUROPEEN**

(45) Date de publication et mention
de la délivrance du brevet:
**19.08.2015 Bulletin 2015/34**

(51) Int Cl.:
**A61B 5/00** *(2006.01)* **G01N 33/487** *(2006.01)*

(21) Numéro de dépôt: **11354077.7**

(22) Date de dépôt: **12.12.2011**

(54) **Dispositif et procédé de détermination d'un débit d'excrétion d'un fluide corporel par un individu ou un animal**

Vorrichtung und Verfahren zur Bestimmung der Ausscheidungsmenge einer Körperflüssigkeit einer Person oder eines Tieres

Device and method for determining an excretion rate of a bodily fluid by a human or an animal

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **14.12.2010 FR 1004864**

(43) Date de publication de la demande:
**20.06.2012 Bulletin 2012/25**

(73) Titulaire: **COMMISSARIAT A L'ENERGIE ATOMIQUE ET AUX ENERGIES ALTERNATIVES**
**75015 Paris (FR)**

(72) Inventeurs:
• **Revol-Cavalier, Frédéric**
**38180 Seyssins (FR)**

• **Lambert, Aurélien**
**74450 Saint Jean de Sixt (FR)**

(74) Mandataire: **Dubreu, Sandrine et al**
**Cabinet Hecké**
**10, rue d'Arménie**
**Europole**
**BP 1537**
**38025 Grenoble Cedex 1 (FR)**

(56) Documents cités:
**WO-A1-02/091917      FR-A1- 2 669 529**
**JP-A- H0 951 877      JP-A- 2010 046 196**
**US-A- 5 050 604      US-A- 5 882 931**

**Description**

**Domaine technique de l'invention**

L'invention concerne un dispositif de détermination d'un débit d'excrétion d'un fluide corporel par un individu ou un animal comportant :

- un élément absorbant le fluide corporel muni d'au moins un couple d'électrodes $C_i$ connecté électriquement à des moyens de mesure d'au moins un paramètre électrique $X_i$ de la partie dudit élément située entre ledit couple d'électrodes $C_i$ et,
- des moyens de séparation supportant ledit élément absorbant, destinés à être mis en contact avec une source d'excrétion du fluide corporel et disposés entre ladite source et ledit élément absorbant, lesdits moyens de séparation comportant un orifice d'entrée du fluide corporel exposant une partie de l'élément absorbant de manière à créer un chemin de diffusion du fluide corporel à travers ledit élément absorbant.

L'invention concerne également un procédé de détermination d'un débit d'excrétion d'un fluide corporel par un individu ou un animal.

**État de la technique**

De nombreux travaux ont montré l'intérêt d'une bonne hydratation chez les individus notamment lors d'activités physiques sportives ou chez les personnes fragiles comme les nourrissons ou les personnes âgées. La perte hydrique due à la transpiration ou le manque d'hydratation peut entraîner l'apparition de troubles physiologiques, par exemple, la perte de poids, des troubles de la concentration, une fatigue importante ou des vertiges. Pour les cas de déshydratation les plus sévères, des pertes de facultés intellectuelles ou de troubles physiologiques pouvant entraîner la mort de l'individu ou de l'animal peuvent également survenir.

Pour éviter la déshydratation ou la surhydratation, une approche consiste à évaluer la perte hydrique par sudation c'est-à-dire la quantité de sueur transpirée sur un temps donné. Cette évaluation est, classiquement, pratiquée par pesée sur une balance différentielle. L'individu est pesé à plusieurs reprises, tout au long d'un exercice physique ou lors du contrôle pour permettre de calculer le rapport entre la perte hydrique et le poids de l'individu. Ce rapport est caractéristique du taux de déshydratation de l'individu et, par conséquent, de son état d'hydratation. Néanmoins, cette solution est précise et sensible, mais ne demeure applicable qu'en laboratoire et ne peut convenir à des applications nomades.

De récents travaux ont proposé des dispositifs permettant d'évaluer la perte hydrique d'un individu à partir de mesure du débit de sueur sécrétée lors d'un exercice physique ou lors d'une crise cardiaque.

À titre d'exemple représenté à la figure 1, le document JP-A-2010046196 décrit un dispositif de mesure de sudation comportant un matériau absorbant 1 la sueur, disposé entre un premier film 2 ayant une fenêtre transparente 3 et second film 4. Le matériau absorbant 1 est muni d'un indicateur coloré 5 changeant de couleur au contact de la sueur. L'indicateur coloré 5 est associé à une échelle graduée permettant de réaliser la correspondance entre la coloration du matériau absorbant 1 et une quantité de sueur sécrétée par le corps humain. Une ouverture 6 est aménagée dans le second film 4 afin d'exposer le matériau absorbant 1 et permettre l'introduction et l'absorption de la sueur au sein du matériau absorbant 1. Le dispositif de mesure de sudation permet de mesurer simplement la quantité de sueur sécrétée, par visualisation de la progression de la sueur teintée par l'indicateur coloré 5, le long du matériau absorbant 1. Néanmoins, l'utilisation d'un tel dispositif de mesure de sudation est limitée aux applications où il est possible de le positionner sur des parties visibles du corps humain. Seule une lecture unique est réalisable à l'issue de l'exercice physique ou de la crise cardiaque lorsque le dispositif de mesure ne peut être appliqué que sur des parties non-visibles.

Or, de nombreuses applications nécessitent un suivi de la sudation en temps réel et ne permettent pas une application du dispositif de mesure uniquement sur des parties visibles du corps d'un individu. Un tel dispositif de mesure est, par exemple, inadapté pour suivre une activité physique intense où la perte hydrique est importante et/ou une activité réalisée dans des conditions de travail particulières comme dans la sécurité civile ou militaire où le port d'une tenue étanche de protection, souvent opaque, contre la radioactivité, les risques chimiques ou biologiques est obligatoire.

Par ailleurs, l'efficacité d'un dispositif de mesure de sudation dépend notamment de son positionnement sur le corps humain car la sueur est sécrétée de façon hétérogène selon la partie du corps humain considérée. La sudation se concentre effectivement sur certaines zones comme le dos et le thorax, habituellement, cachées par un vêtement.

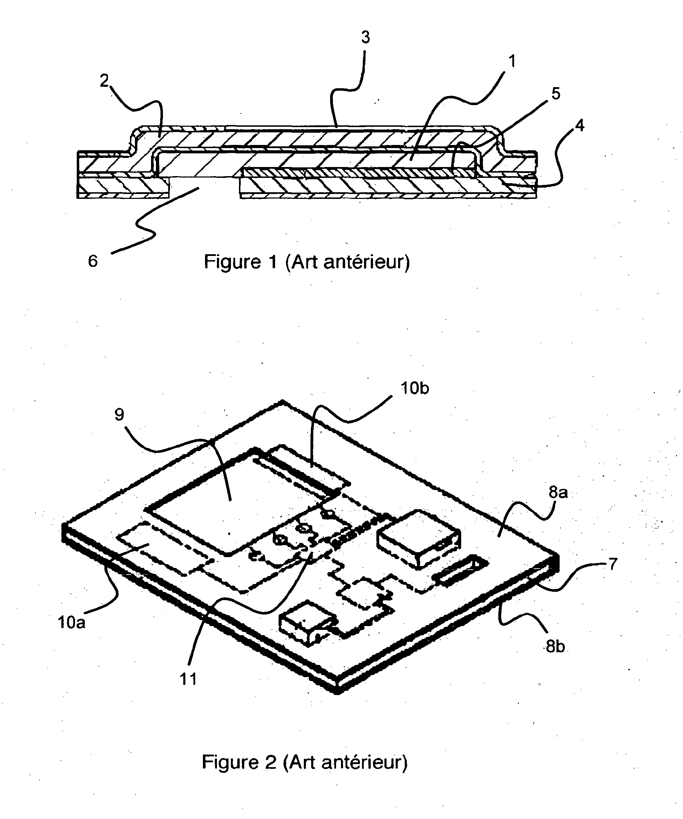

Le document JP-A-9051877 propose un dispositif flexible appliqué sur le corps d'un individu et qui permet de mesurer et d'enregistrer, en temps réel, une quantité de sueur émise. Comme représenté à la figure 2, le dispositif flexible comporte un corps 7 perméable à la sueur, disposé entre deux films plastiques imperméables, 8a et 8b. Un des films 8a est pourvu d'une ouverture 9 et comprend une face adhésive permettant de fixer le dispositif sur la peau de l'individu. Deux électrodes, 10a et 10b, identiques sont disposées dans le corps 7 du dispositif et connectées à un circuit de mesure 11, pour permettre de mesurer des valeurs de la conductivité entre les deux électrodes 10a et 10b. La mesure est basée sur le principe qu'une variation de conductivité du milieu compris entre les deux électrodes, 10a et 10b, est représentative du taux de sécrétion par l'individu et permet de remonter à la mesure du débit de sécrétion. Cependant, une telle interprétation ne tient pas compte d'autres paramètres qui peuvent également affecter la précision de la mesure. En effet, la valeur de la conductivité dépend également de la concentration ionique de la sueur. Or, la concentration ionique de la sueur peut varier indépendamment de la quantité de sueur sécrétée, par exemple, en fonction de l'intensité de l'activité physique ou au cours de la journée. De même, l'évaporation de la sueur en périphérie du dispositif peut, également, créer un gradient de concentration au sein du corps 7 et rendre l'interprétation des résultats difficiles, en faussant les mesures. Enfin, l'utilisation des deux mêmes électrodes, 10a et 10b, pour les mesures successives peut altérer la précision du dispositif au cours du temps.

Le document FR 2 669 529 divulgue un détecteur de présence d'urine. Le capteur comporte des paires d'électrodes rapprochées et des moyens de détection des variations de la résistance électrique de la région comprise dans l'intervalle entre les électrodes lors de la présence d'urine.

**Objet de l'invention**

L'invention a pour but un dispositif, précis et fiable, permettant d'évaluer, rapidement, en temps réel et en continu, un débit d'excrétion d'un fluide corporel par un individu ou un animal.

L'invention a également pour but de proposer un dispositif embarqué facile à utiliser, pour un large champ d'application.

En particulier, l'invention a également pour but un dispositif de détermination d'un débit d'excrétion de la sueur permettant d'évaluer la perte hydrique par sudation de l'individu ou de l'animal.

Selon l'invention, ce but est atteint par les revendications annexées et plus particulièrement par le fait que l'élément absorbant comporte au moins trois d'électrodes espacées les unes des autres et connectées aux moyens de mesure de manière à être couplées électriquement deux à deux et à former au moins deux couples d'électrodes $C_i$ et par le fait qu'au moins deux des électrodes sont des premières électrodes $E^1_i$ placées de façon séquentielle le long du chemin de diffusion de sorte que chacune desdites premières électrodes $E^1_i$ est à une distance $d_i$ de l'orifice d'entrée représentative d'un volume $V_i$ de fluide corporel absorbé par l'élément absorbant.

L'invention a également pour but un procédé simple à mettre en oeuvre et utilisant un tel dispositif permettant de déterminer, de façon précise et reproductible, un taux d'excrétion $Q_i$ d'un fluide corporel par un individu ou un animal pour obtenir un débit d'excrétion global et/ou un débit d'excrétion instantané à partir dudit taux d'excrétion $Q_i$.

**Description sommaire des dessins**

D'autres avantages et caractéristiques ressortiront plus clairement de la description qui va suivre de modes particuliers de réalisation de l'invention donnés à titre d'exemples non-limitatifs et représentés aux dessins annexés, dans lesquels :

- La figure 1 représente, schématiquement et en coupe, un dispositif de mesure de sudation selon l'art antérieur.
- La figure 2 représente, schématiquement et en perspective, un autre dispositif de mesure de sudation selon l'art antérieur.
- Les figures 3 et 4 représentent schématiquement et, respectivement, en coupe et en vue de dessus, un dispositif de détermination d'un débit d'excrétion d'un fluide corporel selon un mode de réalisation particulier de l'invention.
- Les figures 5 à 10 représentent, schématiquement et en coupe, différentes étapes d'un procédé de détermination d'un débit d'excrétion d'un fluide corporel à partir d'un taux d'excrétion du fluide corporel selon l'invention.
- La figure 11 est un graphique représentant l'évolution de la tension mesurée (en V) en fonction du temps (en UA) à partir d'un dispositif selon l'exemple 2.
- Les figures 12 à 14 représentent, schématiquement et en vue de dessus, trois autres modes de réalisation particuliers d'un dispositif selon l'invention.

**Description de modes particuliers de réalisation**

L'invention a pour objet un dispositif permettant de déterminer le taux d'excrétion $Q_i$ ou le débit d'excrétion d'un fluide corporel par un individu ou un animal. Le dispositif est, avantageusement, destiné à une utilisation embarquée sur tout individu ou animal apte à excréter un fluide corporel. L'utilisation du dispositif est réalisée, de préférence, en continu et en temps réel, pour une lecture directe du taux d'excrétion d'un fluide corporel. En fait dans la présente description, le taux d'excrétion servira avantageusement à déterminer un débit d'excrétion d'un fluide corporel qui pourra, par exemple être de type global ou instantané. Ainsi, par la suite lorsque l'on parle de taux, il sera possible de remplacer la notion de taux par la notion de débit.

Le fluide corporel peut, par exemple, être de la sueur ou de l'urine. Le dispositif peut être intégré dans un vêtement pour la détermination du taux de sudation ou dans une couche pour la détermination du taux d'excrétion d'urine ou du débit d'excrétion d'urine.

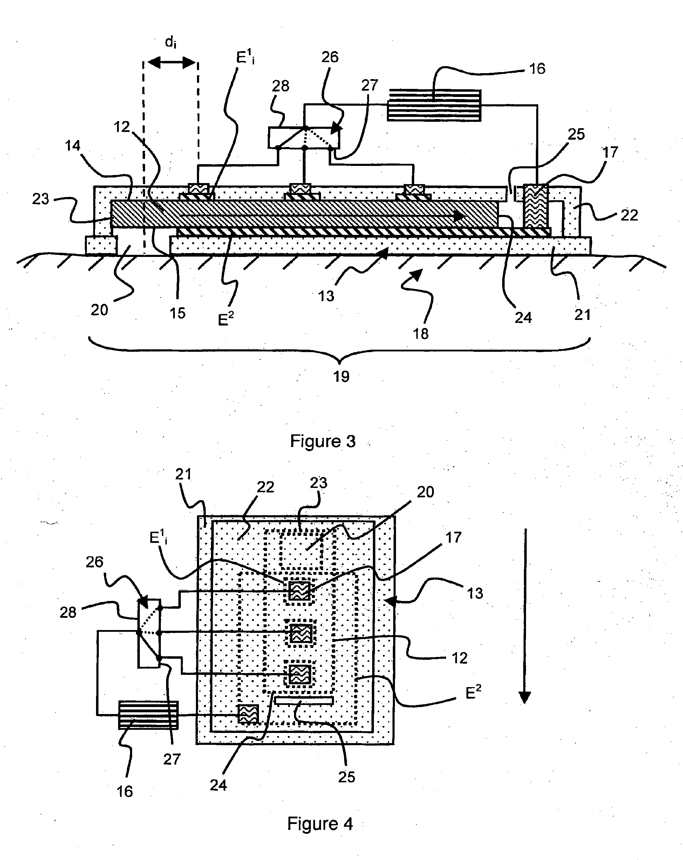

Selon un premier mode de réalisation particulier représenté aux figures 3 et 4, le dispositif de détermination d'un taux d'excrétion $Q_i$ ou d'un débit d'excrétion d'un fluide corporel par un individu ou un animal comporte un élément absorbant 12 le fluide corporel et des moyens de séparation 13 supportant l'élément absorbant 12. Les moyens de séparation 13 sont, de préférence, imperméables. Lorsque l'élément absorbant 12 est disposé le long du corps de l'individu ou de l'animal, les moyens de séparation 13 imperméables supportent l'élément absorbant 12 et assurent une isolation vis-à-vis du corps de l'individu.

L'élément absorbant 12 peut avoir une première face principale 14 et une seconde face principale 15 opposée à ladite première face principale 14. L'élément absorbant 12 peut, avantageusement, être choisi parmi du papier absorbant, un tissu et une partie d'un vêtement.

L'élément absorbant 12 comporte également au moins trois d'électrodes espacées les unes des autres et, avantageusement, formées par un même matériau.

Les électrodes sont connectées électriquement à des moyens de mesure 16 d'au moins un paramètre électrique $X_i$ de manière à être couplées électriquement deux à deux et à former au moins deux couples d'électrodes $C_i$. Des reprises de contact 17 sont prévues sur chaque électrode pour assurer leur connexion électrique aux moyens de mesure 16 électrique.

Les moyens de séparation 13, de préférence, imperméables sont destinés à être mis en contact avec une source d'excrétion du fluide corporel et disposés entre la source d'excrétion du fluide corporel et l'élément absorbant 12. La source d'excrétion du fluide corporel est, par exemple, la peau 18 d'un individu ou d'un animal. Les moyens de séparation 13 peuvent être formés par un matériau imperméable au fluide corporel c'est-à-dire qui empêche le fluide corporel de traverser les moyens de séparation 13 imperméables. Les moyens de séparation 13 sont, par exemple, formés par un matériau plastique choisi parmi le polyéthylène, polystyrène, polyacrylique, polyéthane, polyimide, polyamide, polyesther, silicone, polytétrafluoroéthylène (PTFE) et les fibres creuses, imperméables aux liquides et laissant passer la vapeur d'eau.

Comme représenté à la figure 3, les moyens de séparation 13 ont, en particulier, une partie externe 19 destinée à être appliquée sur la peau 18 qui peut, avantageusement, être recouverte d'un matériau adhésif comme de la colle, pour fixer le dispositif à l'individu ou l'animal et le maintenir en place durant toute la durée des mesures de détermination du taux d'excrétion $Q_i$. Le dispositif peut ainsi être utilisé comme dispositif embarqué.

Les moyens de séparation 13 imperméables comportent un orifice d'entrée 20 du fluide corporel exposant une partie de la seconde face principale 15 de l'élément absorbant 12 de manière à créer un chemin de diffusion du fluide corporel à travers l'élément absorbant 12.

Par chemin de diffusion, on entend le parcours d'un front de diffusion d'un liquide qui se déplace dans l'élément absorbant 12 depuis l'orifice d'entrée 20, et qui s'éloigne dudit orifice d'entrée 20.

Le chemin de diffusion du fluide corporel est, de préférence, unidirectionnel. On entend par "unidirectionnel" le fait que le fluide corporel migre de l'orifice d'entrée 20 vers une même zone, selon une direction spécifique unique représentée par la flèche aux figures 3 et 4.

L'orifice d'entrée 20 est situé, de préférence, au niveau de la partie externe 19 des moyens de séparation 13.

Les moyens de séparation 13 imperméables permettent d'isoler l'élément absorbant 12 de la peau 18 et d'assurer le passage du fluide corporel excrété par la peau 18 à travers l'orifice d'entrée 20.

Les moyens de séparation 13 imperméables sont, avantageusement, constitués par une enveloppe imperméable dans laquelle est logé l'élément absorbant 12.

Comme illustré dans l'exemple des figures 3 et 4, et de manière applicable aux différents modes de réalisation par la suite, les électrodes associées à l'élément absorbant 12 ne sont pas localisées en regard de l'orifice d'entrée 20. Autrement dit, les électrodes sont situées à distance de l'orifice d'entrée 20.

Comme représenté à la figure 3, l'enveloppe imperméable peut, par exemple, être formée par des premier et second films imperméables, respectivement 21 et 22, joints hermétiquement de manière à aménager le logement de l'élément absorbant 12. Les reprises de contact 17 traversent l'enveloppe afin d'assurer la connexion électrique des électrodes avec les moyens de mesure 16 électrique.

La partie externe 19 des moyens de séparation 13 imperméables est, de préférence, située au niveau du premier film imperméable 21. La partie externe 19 peut, par exemple, constituer la face externe du premier film imperméable 21.

L'orifice d'entrée 20 est aménagé dans le premier film imperméable 21 de manière à traverser toute l'épaisseur du premier film imperméable 21.

Comme représenté aux figures 3 et 4, le second film imperméable 22 recouvre, de préférence, entièrement l'élément absorbant 12, notamment, la première face principale 14 pour isoler l'élément absorbant 12 de l'environnement extérieur et, en particulier, le protéger contre l'humidité environnante. Ainsi, l'enveloppe imperméable garantit l'exactitude des mesures et évite toutes erreurs dues aux variations d'hygrométrie ou de température de l'environnement.

L'élément absorbant 12 a, par exemple, une forme de bandelette de longueur, avantageusement, comprise entre 10mm et quelques centaines de millimètres, de préférence, entre 10mm et 100mm et, de largueur, avantageusement, comprise entre quelques millimètres et quelques dizaines de millimètres, de préférence, entre 2mm et 10mm. La bandelette a des première et seconde extrémités, respectivement 23 et 24, correspondant à la largeur de la bandelette. L'orifice d'entrée 20 est situé à proximité de la première extrémité 23, par exemple, à une distance de la première extrémité 23 inférieure à 5% de la longueur de la bandelette. Ainsi, le chemin de diffusion commence à l'orifice d'entrée 20 et se poursuit le long de la bandelette vers la seconde extrémité 24 (flèche aux figures 3 et 4).

L'enveloppe comporte, avantageusement, un orifice de sortie 25 du fluide corporel situé au niveau de la seconde extrémité 24 de l'élément absorbant 12, pour permettre l'évacuation du fluide corporel ayant migré sur tout le chemin de diffusion jusqu'à la seconde extrémité 24. L'orifice de sortie 25 peut être réalisé à travers le second film imperméable 22.

Au moins deux des électrodes sont des premières électrodes $E^1_i$ disposées au niveau de la première face principale 14 de l'élément absorbant 12 et placées de façon séquentielle le long du chemin de diffusion de sorte que chacune des premières électrodes $E^1_i$ est à une distance $d_i$ de l'orifice d'entrée 20.

Comme représenté à la figure 3, les premières électrodes $E^1_i$ sont disposées sur la première face principale 14 de l'élément absorbant 12.

i représente le rang de la première électrode $E^1_i$ dans la séquence formée. Le rang i augmente en passant d'une première électrode $E^1_i$ à une autre première électrode $E^1_{(i+1)}$, adjacente, en s'écartant de l'orifice d'entrée 20.

Pour une meilleure compréhension de l'invention et pour des raisons de clarté, lorsque la description s'appliquera indifféremment à l'une quelconque des premières électrodes $E^1_i$ ou à une de ses caractéristiques, on utilisera dans la suite de la description l'indice i pour identifier l'électrode ou une de ses caractéristiques. En revanche, lorsque la description s'appliquera à une première électrode en particulier ou à une caractéristique spécifique de ladite première électrode, on utilisera à la place de l'indice i, le rang correspondant de la première électrode considérée. Ainsi, on utilisera $E^1_i$ pour identifier l'une quelconque des premières électrodes $E^1_1$, $E^1_2$ ou $E^1_3$ et, $E^1_1$ si l'on souhaite identifier uniquement la première électrode $E^1$ de rang i=1.

Pour chaque première électrode $E^1_i$ la distance $d_i$ répond aux deux conditions (1) et (2) suivantes :

$$d^1_i > 0 \text{ et} \qquad (1)$$

$$d^1_i < d^1_{(i+1)} \qquad (2)$$

Chaque distance $d_i$ est représentative du volume $V_i$ de fluide corporel absorbé par l'élément absorbant 12. La position de la première électrode $E^1_i$ sur le chemin de diffusion est, par conséquent, représentative d'un volume $V_i$ de fluide corporel absorbé par l'élément absorbant 12. Le volume $V_i$ peut, avantageusement, être un volume moyen calculé à partir d'une gamme de volume.

La corrélation entre les volumes $V_i$ et les distances $d_i$ est réalisée classiquement par étalonnage, préalablement à la détermination du taux d'excrétion $Q_i$ de l'individu ou de l'animal. On entend par taux d'excrétion $Q_i$, la quantité de fluide corporel excrétée par l'individu ou l'animal.

En outre, un tableau de correspondance permet de relier chaque volume $V_i$ à un taux d'excrétion $Q_i$ donné. Le taux d'excrétion $Q_i$ peut être obtenu par extrapolation, selon tout procédé connu, en tenant compte éventuellement de plusieurs paramètres caractéristiques de l'individu ou de l'animal, notamment de la zone de la peau 18 sur laquelle est appliqué le dispositif, du sexe, du poids, de la taille et de l'âge de l'individu ou de l'animal.

Comme représenté à la figure 3, le dispositif comporte, de préférence, au moins trois premières électrodes $E^1_i$. Les premières électrodes $E^1_i$ sont, de préférence, disposées en ligne selon la direction du chemin de diffusion (flèche aux figures 3 et 4). Les premières électrodes $E^1_i$ sont disposées entre l'enveloppe et la première face principale 14 de l'élément absorbant 12. Le second film imperméable 22 recouvre les premières électrodes $E^1_i$ disposées sur la première face principale 14 de l'élément absorbant 12.

Au moins une des électrodes est, avantageusement, une seconde électrode $E^2$ disposée entre les moyens de séparation 13 imperméables et la seconde face principale 15 de l'élément absorbant 12.

La seconde électrode $E^2$ est en contact direct avec la seconde face principale 15 de l'élément absorbant 12 de manière à ce que le chemin de diffusion du fluide corporel passe entre la seconde électrode $E^2$ et les premières électrodes $E^1_i$.

Alternativement, au lieu d'être sur l'élément absorbant 12, les premières électrodes $E^1_i$ et/ou la seconde élec-

trode $E^2$ peu(ven)t être disposée(s) dans l'élément absorbant 12 à la condition qu'au moins une partie de l'élément absorbant 12 sépare les premières électrodes $E^1_i$ de la seconde électrode $E^2$. Les premières électrodes $E^1_i$ se trouvent alors au niveau de la première face principale 14 et/ou la seconde électrode $E^2$ se trouve au niveau de la seconde face principale 15.

Les premières électrodes $E^1_i$ et la seconde électrode $E^2$ sont, de préférence, métalliques. Les premières électrodes $E^1_i$ et la seconde électrode $E^2$ sont, par exemple, en cuivre, aluminium, Or, platine, carbone ou oxyde d'indium. Les premières électrodes $E^1_i$ et la seconde électrode $E^2$ peuvent également comporter un polymère conducteur ou un polymère classiquement rendu conducteur par adjonction de particules métalliques telles que des particules d'argent ou d'or ou de particules de carbone.

L'élément absorbant 12 est muni d'au moins deux couples d'électrodes $C_i$ connectés aux moyens de mesure 16. Le dispositif comporte, avantageusement, une unique seconde électrode $E^2$ pouvant être couplée électriquement avec chacune des premières électrodes $E^1_i$, ce qui forme autant de couples d'électrodes $C_i$.

Un commutateur électrique 26, à plusieurs voies, relie les premières électrodes $E^1_i$ aux moyens de mesure 16 électrique et permet de coupler sélectivement chaque première électrode $E^1_i$ à la seconde électrode $E^2$, pour former les couples d'électrodes $C_i$.

Comme représenté aux figures 3 et 4, le commutateur électrique 26 présente des première et seconde bornes, respectivement 27 et 28, permettant trois positions possibles du commutateur électrique 26, les positions 1, 2 et 3. Les premières électrodes $E^1_1$, $E^1_2$ et $E^1_3$ sont reliées à la première borne 27 du commutateur électrique 26. En choisissant la position du commutateur électrique 26, on peut coupler électriquement la seconde électrode $E_2$ avec au moins une des premières électrodes $E^1_i$. De même, on peut coupler successivement chaque première électrode $E^1_i$ à la seconde électrode $E^2$ et former un couple d'électrodes $C_i$, par commutation successive d'une position à une autre. Ainsi, le basculement sur les positions 1, 2 et 3 permet de former, respectivement, les couples d'électrodes $C_1$, $C_2$ et $C_3$, en reliant les premières électrodes $E^1_1$, $E^1_2$ et $E^1_3$ correspondantes aux moyens de mesure 16 électrique.

Les moyens de mesure 16 électrique sont connectés à la seconde électrode $E^2$ et à la seconde borne 28. Les moyens de mesure 16 électrique sont destinés à mesurer une résistance électrique, une conductivité, une tension électrique, une mesure électrochimique ou une mesure de capacité, pour chaque couple d'électrodes $C_i$ formé.

La forme spécifique de l'élément absorbant 12 et la position de l'orifice d'entrée 20, relativement à la position de l'élément absorbant 12, définissent le chemin de diffusion. Le fluide corporel absorbé crée, sur le chemin de diffusion, un front de diffusion 29 passant consécutivement entre chaque couple $C_i$ d'électrodes appariées électriquement.

Le passage du fluide corporel dans l'élément absorbant 12 modifie les caractéristiques électriques $X_i$ de la partie de l'élément absorbant 12 située entre les électrodes d'un couple $C_i$. Les caractéristiques sont, par exemple, la conductivité, la résistance ou la capacité. Ainsi, le suivi de paramètres électriques $X_i$, pour les différents couples d'électrodes $C_i$, permet de déterminer la position du front de diffusion 29 du fluide corporel dans l'élément absorbant 12. En outre, la position du front 29 peut être corrélée avec le taux d'excrétion $Q_i$ de fluide corporel et, avantageusement, le débit d'excrétion instantané $D_i$ et le débit d'excrétion global $D_g$.

De même, selon un principe connu, l'application d'une tension entre la première électrode $E^1_i$ et la seconde électrode $E^2$ d'un couple $C_i$ induit un courant d'hydrolyse du fluide corporel présent dans l'élément absorbant 12 à l'état imbibé, pouvant être détecté et suivi par des mesures électrochimiques.

Par ailleurs, le dispositif comporte classiquement un système de contrôle et d'exploitation de données (non représenté) permettant de collecter et de traiter les données provenant des moyens de mesure 16 électrique et, éventuellement, gérer la position et la vitesse de commutation du commutateur électrique 26. Le système de contrôle et d'exploitation de données peut être un ordinateur, un poste de commande délocalisé, un ordinateur portable de type PDA (en anglais "Personal Digital Assistant"). Le système de contrôle et d'exploitation de données peut, éventuellement, permettre de déclencher une alarme.

Le système de contrôle et d'exploitation de données peut être intégré au dispositif sous la forme d'une carte électronique ou d'un ASIC (en anglais "Application Specific Integrated Component").

Alternativement, le dispositif peut comporter des moyens de transmission de données (non représentés) avec ou sans fil, pour permettre le transfert des données au système de contrôle et d'exploitation de données.

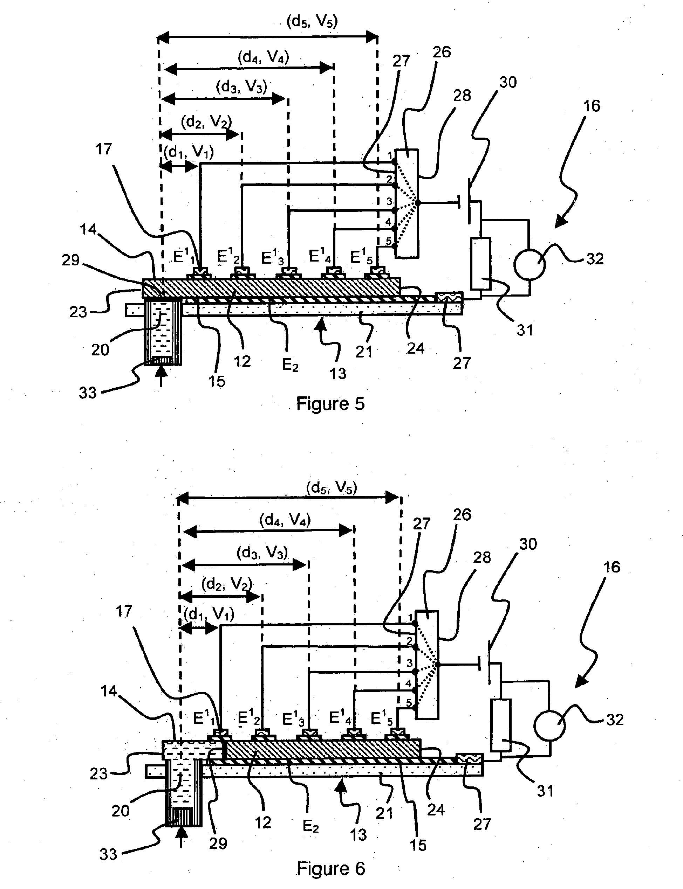

Selon un second mode de réalisation particulier représenté à la figure 5, un dispositif de détermination d'un taux d'excrétion $Q_i$ (ou débit d'excrétion) diffère du premier mode de réalisation décrit ci-dessus en ce qu'il comporte cinq premières électrodes $E^1_1$, $E^1_2$, $E^1_3$, $E^1_4$ et $E^1_5$ formant par couplage électrique avec la seconde électrode $E^2$, respectivement, cinq couples d'électrodes $C_1$, $C_2$, $C_3$, $C_4$ et $C_5$. Ainsi, le commutateur électrique 26 comporte une première borne 27 avec cinq voies reliées aux premières électrodes $E^1_1$, $E^1_2$, $E^1_3$, $E^1_4$ et $E^1_5$ et une seconde borne 28 connectée aux moyens de mesure 16 électrique.

Par ailleurs, le dispositif se distingue de celui décrit ci-dessus en ce que les moyens de séparation 13 imperméables sont uniquement formés par le premier film imperméable 21.

Les moyens de mesure 16 électrique sont constitués par un générateur électrique 30 en série avec une résistance 31. Un voltmètre 32 est monté en parallèle aux bornes de la résistance 31, pour mesurer la tension aux bornes de cette résistance 31. Le couplage électrique de chaque couple d'électrodes $C_1$, $C_2$, $C_3$, $C_4$ ou $C_5$ est effectué par commutation, respectivement, en position 1, 2, 3, 4 ou 5.

Selon un mode de réalisation particulier, un procédé de détermination d'un taux/débit d'excrétion d'un fluide corporel par un individu ou un animal utilisant un dispositif selon le second mode de réalisation décrit ci-dessus comporte une première étape de mise en contact du dispositif avec une source d'excrétion du fluide corporel. La source d'excrétion du fluide corporel est, avantageusement, une zone localisée de la peau 18 de l'individu ou de l'animal.

Le dispositif peut, par exemple, être appliqué sur la zone localisée de la peau 18 de manière à mettre en contact les moyens de séparation 13 imperméables avec la peau 18 et à positionner l'orifice d'entrée 20 en regard de la peau 18. La partie externe 19 du premier film imperméable 21 adhère à la peau 18 sur toute la zone localisée.

La mise en contact est réalisée de manière à assurer le passage du fluide corporel excrété, à travers l'orifice d'entrée 20 vers l'élément absorbant 12. Le fluide corporel excrété par la peau 18 est, par conséquent, canalisé vers l'élément absorbant 12.

Le fluide corporel ainsi introduit dans le dispositif par l'orifice d'entrée 20, se trouve en contact avec l'élément absorbant 12 et imprègne l'élément absorbant 12. Le fluide corporel migre au sein de l'élément absorbant 12 selon le chemin de diffusion, dans le sens de diffusion unidirectionnel (flèche à la figure 5).

Après application du dispositif, on mesure un paramètre électrique $X_i$ pour chaque couple d'électrodes $C_i$.

Le paramètre électrique $X_i$ mesuré est choisi parmi une conductivité, une tension, une résistance, une capacité et/ou une concentration globale en ion.

La série de mesure est réalisée successivement d'un couple d'électrodes $C_i$ au couple d'électrodes $C_{(i+1)}$ adjacent selon l'ordre séquentiel imposé par le chemin de diffusion.

Comme représenté à la figure 5, on mesure successivement $X_i$, $X_2$, $X_3$, $X_4$ et $X_5$ correspondant, respectivement, à chaque couple $C_1$, $C_2$, $C_3$, $C_4$ et $C_5$. La série de mesures peut éventuellement être répétée autant de fois que nécessaire, selon le même ordre séquentiel, afin de réaliser un balayage sur les cinq couples d'électrodes $C_i$.

La série de mesures du paramètre électrique $X_i$ peut être réalisée par commutation successive du commutateur électrique 26 de la position 1 à la position 5, avec une vitesse de commutation déterminée.

La position du front de diffusion 29 dépend du volume $V_i$ de fluide corporel introduit par l'orifice d'entrée 20. Lorsque le front de diffusion 29 franchit une première électrode $E^1_i$, la partie de l'élément absorbant 12 située sous la première électrode $E^1_i$ passe d'un état sec à un état imprégné ce qui entraîne une modification des propriétés conductrices de cette partie. La valeur du paramètre électrique $X_i$ mesuré varie jusqu'à atteindre une valeur seuil $X_{is}$.

La valeur seuil $X_{is}$ est une valeur prédéterminée correspondant au passage du fluide corporel entre les électrodes du couple d'électrodes $C_i$. La valeur seuil $X_{is}$ correspond à la valeur du paramètre électrique $X_i$ obtenue lorsque la partie de l'élément absorbant 12 située entre les électrodes du couple $C_i$ est imbibée de fluide corporel. La valeur seuil $X_{is}$ est typiquement obtenue par étalonnage préalable selon tout procédé connu pour chaque couple d'électrodes $C_i$.

On évalue donc la position d'un front de diffusion 29 du fluide corporel sur le chemin de diffusion relativement à la position des premières électrodes $E^1_i$, par comparaison de la valeur du paramètre électrique $X_i$ mesuré avec la valeur seuil $X_{is}$.

Sachant que la position de chaque première électrode $E^1_i$ est associée à une distance $d_i$ et un volume $V_i$ déterminés, on détermine le taux d'excrétion $Q_i$ à partir de la position du front de diffusion 29 du fluide corporel, par corrélation entre la position du front de diffusion 29 et le volume $V_i$ de fluide corporel absorbé par l'élément absorbant 12.

Le procédé comporte, de préférence, une étape d'identification d'un temps $T_i$ correspondant à l'instant où la valeur du paramètre électrique $X_i$ du couple d'électrodes $C_i$ franchit la valeur seuil $X_{is}$.

Les données du paramètre électrique $X_i$ et du temps $T_i$ sont collectées et transmises selon tout procédé connu au système de contrôle et d'exploitation de données.

L'étape de détermination du taux d'excrétion $Q_i$ peut être suivie d'une étape de calcul d'un débit d'excrétion. Le débit d'excrétion peut être un débit global et/ou un débit d'excrétion instantané. Les débits d'excrétion sont calculés à partir du taux d'excrétion $Q_i$ déterminé et du temps $T_i$ correspondant.

Le débit d'excrétion global ou le débit d'excrétion instantané est calculé à partir d'une durée $\Delta T_{ii'}$. La durée $\Delta T_{ii'}$ correspond au temps nécessaire au fluide corporel pour migrer dans l'élément absorbant 12, d'un couple d'électrodes $C_i$ à un autre couple d'électrodes $C_{i'}$ et parcourir la distance séparant les couples $C_i$ et $C_{i'}$.

La durée $\Delta T_{ii'}$ est calculée à partir des temps $T_i$ et $T_{i'}$ selon l'équation (1) suivante :

$$\Delta T_{ii'} = T_{i'} - T_i \qquad\qquad (1)$$

À titre d'exemple, le débit d'excrétion global Dg et le débit d'excrétion instantané $D_i$ peuvent être calculés par les équations (2) et (3) suivantes :

$$D_g = (Q_5 - Q_1) / (T_5 - T_1) \qquad\qquad (2)$$

$$D_i = (Q_2 - Q_1)/ (T_2 - T_1) \qquad\qquad (3)$$

Le procédé de détermination du taux d'excrétion $Q_i$ (ou débit d'excrétion) peut, avantageusement, être couplé à des mesures de conductivité du fluide corporel absorbé. En appliquant une tension électrique sur chaque couple d'électrodes $C_i$, on peut mesurer la conductivité du fluide corporel. Or, la valeur de la conductivité de la solution permet de suivre la perte sudorale d'un individu lors d'un exercice physique intense. On peut ainsi connaître la concentration en sel perdu par l'individu et compenser cette perte par l'administration d'une quantité adaptée de solution énergétique.

### Exemple 1

Le dispositif est réalisé avec un élément absorbant 12 constitué par une bandelette de papier absorbant commercialisé par la société Wattman sous la référence PS 903. Les électrodes sont en cuivre. Les moyens de mesure 16 sont formés par une pile de 3 V et une résistance 31 de 1 M Ohm.

Le procédé de détermination du taux d'excrétion $Q_i$ (ou du débit d'excrétion en utilisant le taux d'excrétion) est réalisé à partir de la mesure de tensions $U_i$ aux bornes de la résistance 31 en fonction du temps.

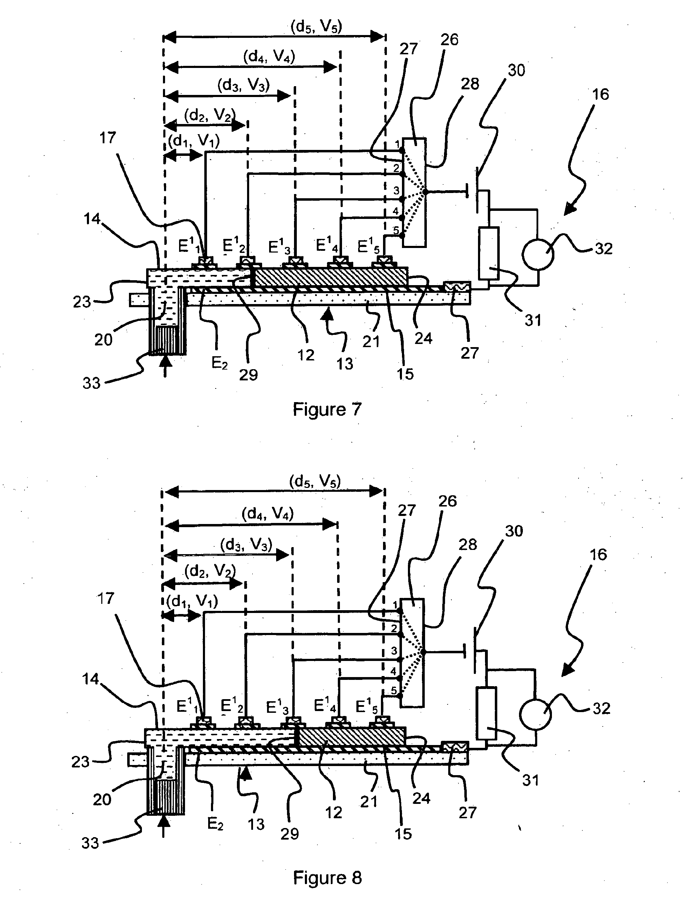

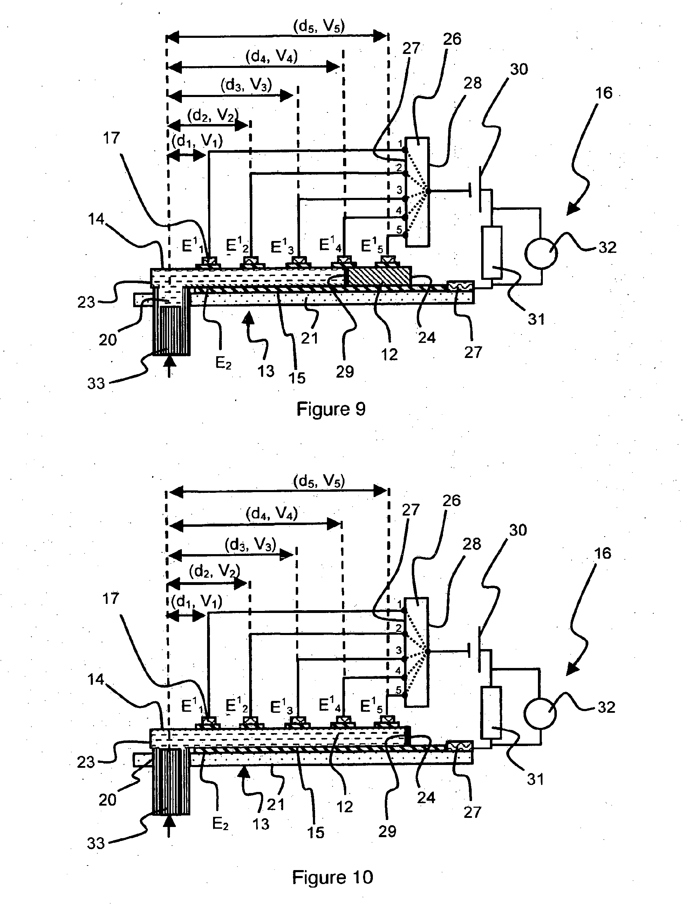

Comme représenté aux figures 5 à 10, la commutation est réalisée, de préférence, successivement d'une position à une autre, de manière à scanner régulièrement les cinq voies associées aux cinq couples d'électrodes $C_1$, $C_2$, $C_3$, $C_4$ et $C_5$. Autrement dit, lorsque la valeur de la tension $U_i$ atteint un certain seuil, le temps $T_i$ correspondant à l'instant de franchissement du seuil est relevé, et le commutateur est activé, de telle sorte que la valeur mesurée devient la tension $U_{i+1}$ associée au couple $C_{i+1}$.

À partir d'un orifice d'entrée 20, on introduit de façon régulière, à l'aide d'un pousse-seringue 33, une solution aqueuse de chlorure de sodium à une concentration de 50mM avec un débit global fixé à 10$\mu$l/min. La solution de NaCl permet de modéliser un fluide corporel comme la sueur. Au fur et à mesure de la progression du front de diffusion 29, l'élément absorbant 12 passe sous chaque première électrode $E^1_i$, le long du chemin de diffusion, d'un état sec à un état imbibé par le fluide corporel.

Les résultats obtenus ont été répertoriés dans le tableau suivant :

| Position du front de diffusion | $C_1$ | $C_2$ | $C_3$ | $C_4$ | $C_5$ |
|---|---|---|---|---|---|
| Figure correspondante | 6 | 7 | 8 | 9 | 10 |
| Volume absorbé lors de la détection ($\mu$l) | 7 | 11.8 | 16.2 | 21.2 | 26.2 |
| Temps $T_i$ (s) | 0 | 29 | 54 | 85 | 115 |
| Débit instantané $D_i$ ($\mu$l/mn) | - | 9.9 | 10.6 | 9.7 | 10 |

À partir des données collectées, on peut également calculer le débit global Dg selon l'équation (2). Le Dg est égal à (Q5-Q1)/(T5-T1) soit : (26,2-7)/[(115-0)/60] soit 10,34 $\mu$l/min. La valeur de tension seuil $U_{is}$ est fixée à 0.7V.

### Exemple 2

Le dispositif est identique au premier exemple ainsi que les conditions de mesure à l'exception du fait que l'on scanne huit voies associées à huit couples d'électrodes $C_1$, $C_2$, $C_3$, $C_4$, $C_5$, $C_6$, $C_7$ et $C_8$ au lieu de cinq.

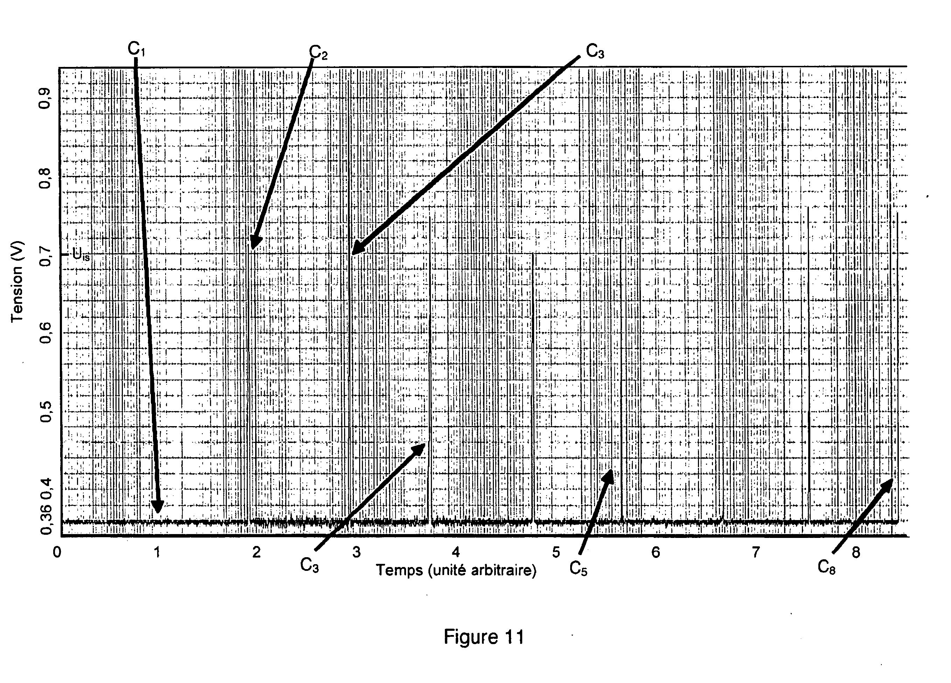

Les résultats obtenus sont représentés à la figure 11 et ont été répertoriés dans le tableau suivant :

| Position du front de diffusion | $C_1$ | $C_2$ | $C_3$ | $C_4$ | $C_5$ | $C_6$ | $C_7$ | $C_8$ |
|---|---|---|---|---|---|---|---|---|
| Volume absorbé lors de la détection ($\mu$l) | 7 | 11.8 | 16.2 | 21.2 | 26.2 | 30.5 | 36.2 | 42 |
| Temps $T_i$ (s) | 0 | 29 | 54 | 85 | 115 | 139 | 174 | 210 |
| Débit instantané $D_i$ ($\mu$l/mn) | - | 9.9 | 10.6 | 9.7 | 10 | 10.7 | 9.8 | 9.7 |

À partir des données collectées, on calcule le débit global Dg selon l'équation (2). Le Dg est égal à (Q8-Q1)/(T8-T1) soit : (42-7)/[(210-0)/60] soit 10$\mu$l/min. La valeur de tension seuil $U_{is}$ est fixée à 0.7V.

Selon une variante non représentée, l'élément absorbant 12 comporte des moyens d'augmentation de la conductivité du fluide corporel. Les moyens d'augmentation de la conductivité peuvent être une encre conductrice, présente dans l'élément absorbant 12 et située entre au moins les électrodes d'un couple d'électrodes $C_i$. Lorsque le fluide corporel atteint l'encre conductrice, elle se dissout ou se disperse, augmentant ainsi la conductivité de l'élément absorbant 12. Le dispositif comportant une telle encre présente une sensibilité plus élevée pour la mesure du paramètre électrique $X_i$ et, par conséquent, permet de déterminer le taux d'excrétion $Q_i$ avec une plus grande précision.

Selon une autre variante non représentée, l'élément absorbant 12 comporte un révélateur colorimétrique de la présence du fluide corporel. Selon un principe similaire à la variante précédente, on peut avantageusement introduire dans l'élément absorbant 12, entre au moins les électrodes d'un couple d'électrodes $C_i$, un révélateur colorimétrique qui au contact du fluide corporel se colore. On peut, par exemple, utiliser une encre non conductrice dans l'élément absorbant 12. Ainsi, un suivi visuel facile et rapide peut, avantageusement, compléter les fonctions du dispositif.

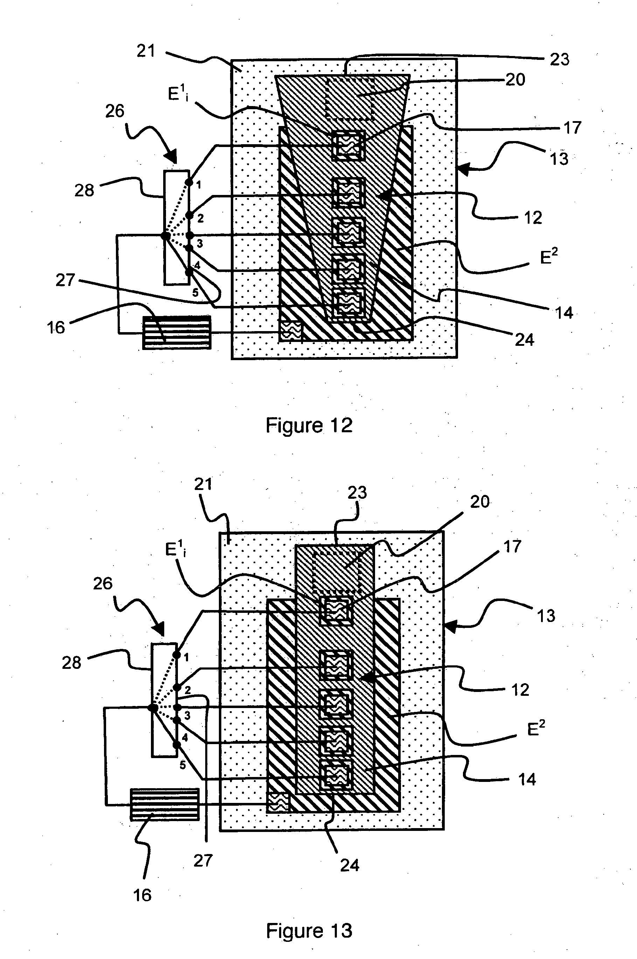

Selon un troisième mode de réalisation représenté à la figure 12, le dispositif est identique au second mode de réalisation à l'exception du fait que l'élément absorbant 12 a une forme de bandelette à section tronconique ayant une grande base et une petite base. L'orifice d'entrée 20 est situé à proximité de la grande base de sorte que le fluide corporel absorbé converge vers la petite base. La forme particulière tronconique de l'élément absorbant 12 compense la perte de vitesse de migration observée le long du chemin de migration, en s'éloignant de l'orifice d'entrée 20. La forme de la bandelette permet de palier à cet inconvénient, en favorisant la migration du fluide corporel au sein de l'élément absorbant 12.

Selon un quatrième troisième mode de réalisation représenté à la figure 13, le dispositif est identique au second mode de réalisation à l'exception du fait que la distance qui sépare deux premières électrodes adjacentes, respectivement $E^1_i$ et $E^1_{(i+1)}$, diminue en s'écartant de l'orifice d'entrée 20. Comme précédemment, cette caractéristique distinctive à pour but de palier à la perte de vitesse de migration du fluide corporel le long du chemin de diffusion.

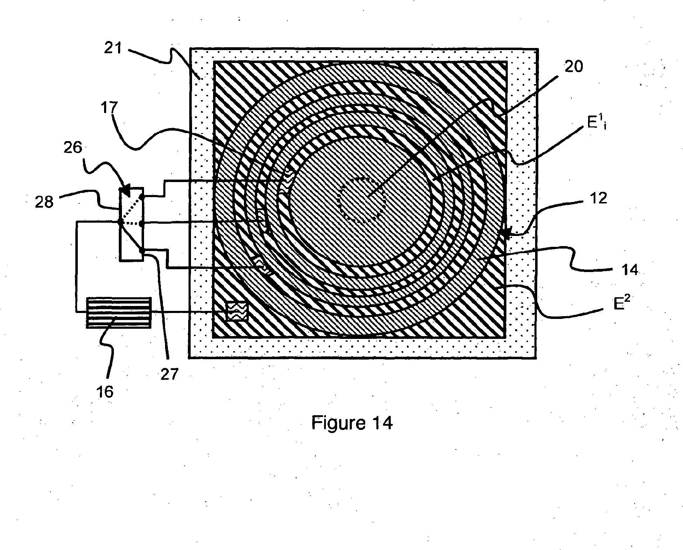

Selon un cinquième mode de réalisation représenté à la figure 14, le dispositif est identique au second mode de réalisation à l'exception du fait que le chemin de diffusion du fluide corporel est concentrique en partant de l'orifice d'entrée 20. Les premières électrodes $E^1_i$ sont des électrodes circulaires formant sur la première face principale 14 de l'élément absorbant 12 des cercles concentriques ayant pour centre, l'orifice d'entrée 20 et s'écartant successivement de l'orifice d'entrée 20.

Bien que les exemples décrits ci-dessus concernent uniquement des dispositifs de détermination du taux d'excrétion $Q_i$ (ou du débit d'excrétion) d'un fluide corporel, comprenant une unique seconde électrode $E_2$, l'invention n'est pas pour autant limitée à ces modes de réalisation qui ne sont décrits qu'à titre illustratif et ne peuvent limiter la portée de l'invention. Ainsi, il peut être envisagé de réaliser un dispositif identique à ceux décrits précédemment mais comportant plusieurs secondes électrodes $E^2_i$. Les secondes électrodes $E^2_i$ sont alors disposées en ligne selon une séquence identique à celle des premières électrodes $E^1_i$ et parallèlement à ladite séquence. L'homme de l'art est en mesure d'adapter la connexion électrique pour permettre le couplage deux à deux des premières et secondes électrodes, respectivement $E^1_i$ et $E^2_i$ pour permettre de suivre le front de diffusion 29 du fluide corporel par mesure d'un paramètre électrique $X_i$ selon le même principe que celui décrit précédemment.

De même, il peut être envisagé de réaliser un dispositif qui ne comporte pas de seconde électrode $E_2$. Les premières électrodes $E^1_1$, sont alors couplées deux à deux selon un schéma électrique approprié pour former les couples d'électrodes $C_i$. Le taux d'excrétion $Q_i$ est déterminé selon un procédé de détermination identique à celui décrit précédemment.

Le dispositif selon l'invention est particulièrement adapté pour déterminer le taux de sudation d'un individu ou d'un animal. Le dispositif ainsi que le procédé utilisant un tel dispositif peuvent, avantageusement, être utilisés pour évaluer la perte hydrique de l'individu ou de l'animal par sudation, à partir de la détermination du taux d'excrétion $Q_i$ qui correspond dans ce cas à un taux de sudation. On entend par sudation la quantité de sueur excrétée par sécrétion de la peau d'un individu ou d'un animal. Le dispositif peut être utilisé par des sportifs ayant des activités physiques intenses nécessitant un suivi du taux de sudation, pour contrôler la déshydratation du sportif. De même, le dispositif peut être intégré dans une combinaison de protection de travailleurs, de pompiers ou de militaires.

Enfin, le dispositif peut être intégré dans une couche pour permettre d'évaluer le taux de remplissage de la couche. Ainsi, le dispositif trouve également une application dans le domaine du suivi et du contrôle de l'incontinence des personnes âgées ou le remplacement des changes d'un nourrisson ou d'un bébé.

Contrairement aux dispositifs de l'art antérieur, le dispositif selon l'invention permet de déterminer de façon reproductible, précise et fiable le taux d'excrétion $Q_i$ d'un fluide corporel par un individu ou un animal. En outre, le dispositif selon l'invention présente l'avantage d'utiliser un couple d'électrodes $C_i$ différent pour chaque mesure, évitant ainsi des imprécisions dues à des mesures successives sur une même électrode. Le procédé utilisant un tel dispositif permet de suivre, en temps réel, l'excrétion d'un fluide corporel d'un individu ou d'un animal, en fournissant un taux d'excrétion $Q_i$ et un débit global ou instantané du fluide corporel. En outre, la détermination du taux d'excrétion $Q_i$ selon l'invention ne dépend pas de la composition du fluide corporel et n'est, par conséquent, pas faussée par une variation de la concentration du fluide corporel au cours du temps, fréquemment observée, en particulier, pour la sueur. Enfin, le procédé selon l'invention permet de coupler la détermination du taux d'excrétion $Q_i$ et du débit global ou instantané à des mesures de conductivité du fluide corporel.

**Revendications**

1. Dispositif de détermination d'un débit d'excrétion d'un fluide corporel par un individu ou un animal, comportant :

   - un élément absorbant (12) le fluide corporel et ayant une première face principale (14) et une seconde face principale (15) opposée à ladite première face principale (14), une partie de ladite seconde face principale (15) étant exposée par l'orifice d'entrée (20),
   - des moyens de séparation (13) supportant ledit élément absorbant (12), destinés à être mis en contact avec une source d'excrétion du fluide corporel et disposés entre ladite source et ledit élément absorbant (12), lesdits moyens de séparation (13) comportant un orifice d'entrée (20) du fluide corporel exposant une partie de l'élément absorbant (12) de manière à créer un chemin de diffusion du fluide corporel à travers ledit élément absorbant (12),

   **caractérisé en ce que** l'élément absorbant (12) est muni d'au moins un couple d'électrodes $C_i$ connecté électriquement à des moyens de mesure (16) d'au moins un paramètre électrique $X_i$ de la partie dudit élément (12) située entre ledit couple d'électrodes $C_i$, **en ce que** l'élément absorbant (12) comporte au moins trois électrodes espacées les unes des autres et connectées aux moyens de mesure (16) de manière à être couplées électriquement deux à deux et à former au moins deux couples d'électrodes $C_i$ et **en ce qu'**au moins deux des électrodes sont des premières électrodes $E^1_i$ placées de façon séquentielle le long du chemin de diffusion de sorte que chacune desdites premières électrodes $E^1_i$ est à une distance $d_i$ de l'orifice d'entrée (20) représentative d'un volume $V_i$ de fluide corporel absorbé par l'élément absorbant (12),
   **en ce que** la forme spécifique de l'élément absorbant (12) et la position de l'orifice d'entrée (20), relativement à la position de l'élément absorbant (12), définissent un chemin de diffusion du fluide corporel à travers ledit élément absorbant (12), et **en ce qu'**au moins une des électrodes est une seconde électrode $E^2$ disposée entre les moyens de séparation (13) et la seconde face principale (15) de l'élément absorbant (12), en contact direct avec la seconde face principale (15) de l'élément absorbant (12) de manière à ce que le chemin de diffusion du fluide corporel passe entre la seconde électrode $E^2$ et les premières électrodes $E^1_i$, ladite seconde électrode $E_2$ étant couplée électriquement avec au moins une desdites premières électrodes $E^1_i$.

2. Dispositif selon la revendication 1, **caractérisé en ce que** les moyens de séparation (13) Imperméables sont constitués par une enveloppe imperméable dans laquelle est logé l'élément absorbant (12) et **en ce que** les premières électrodes $E^1_i$ sont disposées entre l'enveloppe et l'élément absorbant (12).

3. Dispositif selon l'une des revendications 1 et 2, **caractérisé en ce qu'**il comporte au moins trois premières électrodes $E^1_i$.

4. Dispositif selon l'une quelconque des revendications 1 à 3, **caractérisé en ce qu'**il comporte une unique seconde électrode $E^2$ couplée électriquement avec chacune desdites premières électrodes $E^1_i$.

5. Dispositif selon l'une quelconque des revendications 1 à 4, **caractérisé en ce que** le chemin de diffusion du fluide corporel est concentrique en partant de l'orifice d'entrée (20) et **en ce que** les premières électrodes $E^i_l$ sont des électrodes circulaires formant sur de l'élément absorbant (12) des cercles concentriques ayant pour centre l'orifice d'entrée (20) et s'écartant successivement dudit orifice (20).

6. Dispositif selon l'une quelconque des revendications 1 à 5, **caractérisé en ce que** le chemin de diffusion du fluide corporel est unidirectionnel et **en ce que** les premières électrodes $E^1_i$ sont disposées en ligne.

7. Dispositif selon la revendication 6, **caractérisé en ce que** l'élément absorbant (12) a une forme de bandelette à section tronconique ayant une grande base et une petite base et **en ce que** l'orifice d'entrée (20) est situé à proximité de la grande base de sorte que le fluide corporel absorbé converge vers la petite base.

8. Dispositif selon l'une des revendications 6 et 7, **caractérisé en ce que** la distance séparant deux premières électrodes adjacentes, respectivement $E^1_i$ et $E^1_{(i+1)}$, diminue en s'écartant de l'orifice d'entrée (20).

9. Dispositif selon l'une quelconque des revendications 1 à 8, **caractérisé en ce que** l'élément absorbant (12) est choisi parmi du papier absorbant, un tissu et une partie d'un vêtement.

10. Dispositif selon l'une quelconque des revendications 1 à 9, **caractérisé en ce que** les premières électrodes $E^1_i$ et la seconde électrode $E^2$ sont métalliques.

11. Dispositif selon l'une quelconque des revendications 1 à 10, **caractérisé en ce que** l'élément absorbant (12) comporte des moyens d'augmentation de la conductivité du fluide corporel.

12. Dispositif selon l'une quelconque des revendications 1 à 11, **caractérisé en ce que** l'élément absorbant (12) comporte un révélateur colorimétrique de la présence du fluide corporel.

13. Procédé de détermination d'un débit d'excrétion d'un fluide corporel par un individu ou un animal, **caractérisé en ce qu'**il comporte les étapes suivantes:

- mise en contact d'un dispositif selon l'une quelconque des revendications 1 à 12, avec une source d'excrétion du fluide corporel de manière à positionner l'orifice d'entrée (20) en regard de ladite source et à assurer le passage du fluide corporel excrété à travers l'orifice d'entrée (20) vers l'élément absorbant (12),
- mesure d'un paramètre électrique $X_i$ pour chaque couple d'électrodes $C_i$, ladite mesure étant réalisée successivement d'un couple d'électrodes $C_i$ au couple d'électrodes $C_{(i+1)}$ adjacent selon un ordre imposé par le chemin de diffusion,
- évaluation de la position d'un front de diffusion (29) du fluide corporel sur le chemin de diffusion, relativement à la position des premières électrodes $E^1_i$, par comparaison de la valeur du paramètre électrique $X_i$ mesuré avec une valeur seuil $X_{is}$ prédéterminée correspondant au passage du fluide corporel entre les électrodes du couple d'électrodes $C_i$ et,
- détermination du taux d'excrétion $Q_i$ à partir de la position du front de diffusion (29), par corrélation entre ladite position et un volume $V_i$ de fluide corporel absorbé par l'élément absorbant (12),
- identification d'un temps $T_i$ correspondant à l'instant où la valeur du paramètre électrique $X_i$ du couple d'électrodes $C_i$ franchit la valeur seuil $X_{is}$,
- détermination du débit d'excrétion global et/ou du débit d'excrétion instantané à partir dudit taux d'excrétion $Q_I$ et du temps $T_i$ correspondant.

14. Procédé selon la revendication 13, **caractérisé en ce que** le débit d'excrétion global ou le débit d'excrétion instantané est calculé à partir d'une durée $\Delta T_{ii'}$ nécessaire au fluide corporel pour migrer dans l'élément absorbant (12), d'un couple d'électrodes $C_i$ à un autre couple d'électrodes $C_{i'}$ et parcourir la distance séparant lesdits couples $C_i$ et $C_{i'}$, ladite durée $\Delta T_{ii'}$ étant égale à la différence des temps $T_{i'} - T_i$.

15. Procédé selon l'une quelconque des revendications 13 à 14, **caractérisé en ce que** le paramètre électrique $X_i$ mesuré est choisi parmi une conductivité, une tension, une résistance, une capacité et/ou une concentration globale en ion.


**Patentansprüche**

1. Vorrichtung zur Bestimmung einer Ausscheidungsmenge einer Körperflüssigkeit durch einen Menschen oder ein Tier, umfassend:

- ein die Körperflüssigkeit absorbierendes Element (12),
- das absorbierende Element (12) tragende Trennmittel (13), die dazu bestimmt sind, mit einer Ausscheidungsquelle der Körperflüssigkeit in Kontakt gebracht zu werden, und die zwischen der Quelle und dem absorbierenden Element (12) angeordnet sind, wobei die Trennmittel (13) eine Öffnung für den Eintritt (20) der Körperflüssigkeit umfassen, die einen Teil des absorbierenden Elements (12) offen legt, um einen Diffusionsweg der Körperflüssigkeit durch das absorbierende Element (12) zu schaffen,

**dadurch gekennzeichnet, dass** das absorbierende Element (12) mit wenigstens einem Elektrodenpaar $C_i$, das mit Mitteln zur Messung (16) wenigstens eines elektrischen Parameters $X_i$ des zwischen dem Elektrodenpaar $C_i$ gelegenen Teils des Elements (12) elektrisch verbunden ist, ausgestattet ist und eine erste Hauptseite (14) sowie eine von der ersten Hauptseite (14) abgewandte zweite Hauptseite (15) aufweist, wobei ein Teil der zweiten Hauptseite (15) durch die Eintrittsöffnung (20) offen gelegt ist,

dass das absorbierende Element (12) wenigstens drei Elektroden umfasst, die voneinander beabstandet und mit den Messmitteln (16) verbunden sind, um paarweise elektrisch gekoppelt zu sein und um wenigstens zwei Elektrodenpaare $C_i$ zu bilden, und dass wenigstens zwei der Elektroden erste Elektroden $E^1_i$ sind, die entlang des Diffusionsweges sequentiell angeordnet sind, so dass eine jede der ersten Elektroden E\ sich in einem Abstand $d_i$ von der Eintrittsöffnung (20) befindet, der für ein durch das absorbierende Element (12) absorbiertes Körperflüssigkeitsvolumen $V_i$ repräsentativ ist,

dass die spezifische Form des absorbierenden Elements (12) und die Position der Eintrittsöffnung (20) relativ zu der Position des absorbierenden Elements (12) einen Diffusionsweg der Körperflüssigkeit durch das absorbierende Element (12) definieren,

und dass wenigstens eine der Elektroden eine zweite Elektrode $E^2$ ist, die zwischen den Trennmitteln (13) und der zweiten Hauptseite (15) des absorbierenden Elements (12), in direktem Kontakt mit der zweiten Hauptseite (15) des absorbierenden Elements (12) angeordnet ist, so dass der Diffusionsweg der Körperflüssigkeit zwischen der zweiten Elektrode $E^2$ und den ersten Elektroden $E^1_i$ verläuft, wobei die zweite Elektrode $E^2$ mit wenigstens einer der ersten Elektroden $E^1_i$ elektrisch gekoppelt ist.

2. Vorrichtung nach Anspruch 1, **dadurch gekennzeichnet, dass** die undurchlässigen Trennmittel (13) durch eine undurchlässige Hülle gebildet sind, in der das absorbierende Element (12) untergebracht ist, und dass die ersten Elektroden $E^1_i$ zwischen der Hülle und dem absorbierenden Element (12) angeordnet sind.

3. Vorrichtung nach einem der Ansprüche 1 und 2, **dadurch gekennzeichnet, dass** sie wenigstens drei erste Elektroden $E^1_i$ umfasst.

4. Vorrichtung nach einem der Ansprüche 1 bis 3, **dadurch gekennzeichnet, dass** sie eine einzige zweite Elektrode $E^2$, die mit jeder der ersten Elektroden $E^1_i$ elektrisch gekoppelt ist, umfasst.

5. Vorrichtung nach einem der Ansprüche 1 bis 4, **dadurch gekennzeichnet, dass** der Diffusionsweg der Körperflüssigkeit ausgehend von der Eintrittsöffnung (20) konzentrisch ist und dass die ersten Elektroden $E^1_i$ kreisförmige Elektroden sind, die an dem absorbierenden Element (12) konzentrische Kreise bilden, deren Mittelpunkt die Eintrittsöffnung (20) ist und die sich von der Öffnung (20) sukzessive entfernen.

6. Vorrichtung nach einem der Ansprüche 1 bis 5, **dadurch gekennzeichnet, dass** der Diffusionsweg der Körperflüssigkeit unidirektional ist und dass die ersten Elektroden $E^1_i$ in einer Reihe angeordnet sind.

7. Vorrichtung nach Anspruch 6, **dadurch gekennzeichnet, dass** das absorbierende Element (12) eine Streifenform mit kegelstumpfförmigem Querschnitt, mit einer großen Basis und einer kleinen Basis aufweist und dass die Eintrittsöffnung (20) in der Nähe der großen Basis gelegen ist, so dass die absorbierte Körperflüssigkeit in Richtung auf die kleine Basis zusammenläuft.

8. Vorrichtung nach einem der Ansprüche 6 und 7, **dadurch gekennzeichnet, dass** der Abstand zwischen zwei benachbarten ersten Elektroden, $E^1_i$ bzw. $E^1_{(i+1)}$, mit zunehmender Entfernung von der Eintrittsöffnung (20) abnimmt.

9. Vorrichtung nach einem der Ansprüche 1 bis 8, **dadurch gekennzeichnet, dass** das absorbierende Element (12) aus absorbierendem Papier, einem Gewebe oder einem Teil eines Kleidungsstücks ausgewählt ist.

10. Vorrichtung nach einem der Ansprüche 1 bis 9, **dadurch gekennzeichnet, dass** die ersten Elektroden $E^1_i$ und die zweite Elektrode $E^2$ metallisch sind.

11. Vorrichtung nach einem der Ansprüche 1 bis 10, **dadurch gekennzeichnet, dass** das absorbierende Element (12) Mittel zur Erhöhung der Leitfähigkeit der Körperflüssigkeit umfasst.

12. Vorrichtung nach einem der Ansprüche 1 bis 11, **dadurch gekennzeichnet, dass** das absorbierende Element (12) einen Farbindikator für das Vorliegen der Körperflüssigkeit umfasst.

13. Verfahren zur Bestimmung einer Ausscheidungsmenge einer Körperflüssigkeit durch einen Menschen oder ein Tier, **dadurch gekennzeichnet, dass** es die folgenden Schritte umfasst:

- Inkontaktbringen einer Vorrichtung nach einem der Ansprüche 1 bis 12 mit einer Ausscheidungsquelle der Körperflüssigkeit derart, dass die Eintrittsöffnung (20) gegenüber der Quelle positioniert ist und dass der Durchgang der ausgeschiedenen Körperflüssigkeit durch die Eintrittsöffnung (20) zu dem absorbierenden Element (12) sichergestellt ist,

- Messen eines elektrischen Parameters $X_i$ für jedes Elektrodenpaar $C_i$, wobei die Messung nacheinander von einem Elektrodenpaar $C_i$ zum benachbarten Elektrodenpaar $C_{(i+1)}$ in einer durch den Diffusionsweg auferlegten Reihenfolge vollzogen wird,

- Auswerten der Position einer Diffusionsfront (29) der Körperflüssigkeit auf dem Diffusionsweg relativ zu der Position der ersten Elektroden $E^1{}_i$, durch Vergleich des Wertes des gemessenen elektrischen Parameters $X_i$ mit einem vorbestimmten Schwellenwert $X_{is}$, der dem Durchgang der Körperflüssigkeit zwischen den Elektroden des Elektrodenpaars $C_i$ entspricht, und

- Bestimmen der Ausscheidungsrate $Q_i$ anhand der Position der Diffusionsfront (29) durch Korrelation zwischen der Position und einem durch das absorbierende Element (12) absorbierten Körperflüssigkeitsvolumen $V_i$,

- Feststellen eines Zeitpunkts $T_i$, der dem Augenblick entspricht, zu dem der Wert des elektrischen Parameters $X_i$ des Elektrodenpaars $C_i$ den Schwellenwert $X_{is}$ überschreitet,

- Bestimmen der Gesamtausscheidungsmenge und/oder der momentanen Ausscheidungsmenge anhand der Ausscheidungsrate $Q_i$ und des entsprechenden Zeitpunkts $T_i$.

14. Verfahren nach Anspruch 13, **dadurch gekennzeichnet, dass** die Gesamtausscheidungsmenge oder die momentane Ausscheidungsmenge anhand einer Dauer $\Delta T_{il'}$ berechnet wird, die die Körperflüssigkeit benötigt, um in dem absorbierenden Element (12) von einem Elektrodenpaar $C_i$ zu einem anderen Elektrodenpaar $C_{i'}$ zu wandern und um die Strecke zwischen den Paaren $C_i$ und $C_{i'}$ zurückzulegen, wobei die Dauer $\Delta T_{il'}$ gleich der Differenz aus den Zeitpunkten $T_{i'} - T_i$ ist.

15. Verfahren nach einem der Ansprüche 13 bis 14, **dadurch gekennzeichnet, dass** der gemessene elektrische Parameter $X_i$ aus einer Leitfähigkeit, einer Spannung, einem Widerstand, einer Kapazität und/oder einer Gesamtionenkonzentration ausgewählt ist.

## Claims

1. A device for determining an excretion flow rate of a body fluid of a person or an animal comprising:

   - an element (12) absorbing the body fluid, and having a first main surface (14) and a second main surface (15) opposite to said first main surface (14), a part of said second main surface (15) being exposed by an inlet opening (20),
   - separating means (13) supporting said absorbent element (12), designed to be placed in contact with a source of excretion of the body fluid and arranged between said source and said absorbent element (12), said separating means (13) comprising an inlet opening (20) of the body fluid exposing a part of the absorbent element (12) so as to create a diffusion path of the body fluid through said absorbent element (12),

   **characterized in that** the absorbent element (12) is equipped with at least one pair of electrodes $C_i$ electrically connected to measuring means (16) for measuring at least one electric parameter $X_i$ of the part of said absorbent element (12) situated between said pair of electrodes $C_i$ and,
   **in that** the absorbent element (12) comprises at least three electrodes spaced apart from one another and connected to the measuring means (16) so as to be electrically coupled two by two and to form at least two pairs of electrodes $C_i$ and **in that** at least two of the electrodes are first electrodes $E^1{}_i$ placed in sequential manner along the diffusion path so that each of said first electrodes $E^1{}_i$ is at a distance from the inlet opening (20) representative of a volume $V_i$ of body fluid absorbed by the absorbent element (12),
   **in that** the specific shape of the absorbent element (12) and the position of the inlet opening (20), relatively to the position of absorbent element (12), define a diffusion path of the body fluid through said absorbent element (12), and **in that** at least one of the electrodes is a second electrode $E^2$ arranged between the separating means (13) and the second main surface (15) of the absorbent element (12), in direct contact with the second main surface (15) of the absorbent element (12) so that the diffusion path of the body fluid passes between the second electrode $E^2$ and the first electrodes $E^1{}_i$, said second electrode $E^2$ being electrically coupled with at least one of said first electrodes $E^1{}_i$.

2. The device according to claim 1, **characterized in that** the impermeable separating means (13) are formed by an impermeable enclosure in which the absorbent element (12) is housed and **in that** the first electrodes $E^1{}_i$ are arranged between the enclosure and the absorbent element (12).

3.  The device according to one of the claims 1 to 2, **characterized in that** it comprises at least three first electrodes $E^1_i$.

4.  The device according to any one of claims 1 to 3, **characterized in that** it comprises a single second electrode $E^2$ electrically coupled with each of said first electrodes $E^1_i$.

5.  The device according to any one of claims 1 to 4, **characterized in that** the diffusion path of the body fluid is concentric starting from the inlet opening (20) and **in that** the first electrodes $E^1_i$ are circular electrodes forming concentric circles on the absorbent element (12), the centre of the circles being formed by the inlet opening (20), and then moving successively away from said opening (20).

6.  The device according to any one of claims 1 to 5, **characterized in that** the diffusion path of the body fluid is unidirectional and **in that** the first electrodes $E^1_i$ are arranged in line.

7.  The device according to claim 6, **characterized in that** the absorbent element (12) is in the form of a strip with a truncated cone-shaped cross-section having a large base and a small base and **in that** the inlet opening (20) is situated in proximity to the large base so that the absorbed body fluid converges towards the small base.

8.  The device according to one of claim 6 to 7, **characterized in that** the distance separating two adjacent first electrodes, respectively $E^1_i$ and $E^1_{(i+1)}$, decreases when moving away from the inlet opening (20).

9.  The device according to one of the claims 1 to 8, **characterized in that** the absorbent element is chosen from absorbent paper, a fabric and a part of an item of clothing.

10. The device according to any one of claims 1 to 9, **characterized in that** the first electrodes $E^1_i$ and the second electrode $E^2$ are made from metal.

11. The device according to one of the claims 1 to 10, **characterized in that** the absorbent element (12) comprises elements for increasing the conductivity of the body fluid.

12. The device according to one of the claims 1 to 11, **characterized in that** the absorbent element (12) comprises a colorimetric developer of the presence of the body fluid.

13. A method for determining an excretion flow rate of a body fluid of a person or an animal, comprising the following steps:

    - bringing a device according to any one of claim 1 to 12, into contact with a source of excretion of the body fluid so as to position the inlet opening (20) facing said source and to ensure flow of the excreted body fluid through the inlet opening (20) to the absorbent element (12),
    - measurement of an electric parameter $X_i$ for each pair of electrodes $C_i$, said measurement being successively performed from one pair of electrodes $C_i$ to the adjacent pair of electrodes $C_{(i+1)}$ in an order imposed by the diffusion path,
    - evaluation of the position of a diffusion front (29) of the body fluid on the diffusion path, relatively to the position of the first electrodes $E_i$, by comparing the value of the measured electric parameter $X_i$ with a predefined threshold $X_{is}$ value corresponding to flow of the body fluid between the electrodes of the pair of electrodes $C_i$ and,
    - determination of an excretion level $Q_i$ from the position of the diffusion front (29) by correlation between said position and a volume $V_i$ of body fluid absorbed by the absorbent element (12),
    - identification of a time $T_i$ corresponding to the moment when the value of the electric parameter $X_i$ of the pair of electrodes $C_i$ reaches the threshold value $X_{is}$,
    - determination of the global excretion flow rate and/or of the instantaneous excretion flow rate from said excretion level $Q_i$ and from the corresponding time $T_i$.

14. The method according to claim 13, **characterized in that** the global excretion flow rate or the instantaneous excretion flow rate is computed from a time period $\Delta T_{ii'}$ necessary for the body fluid to migrate into the absorbent element (12) from one pair of electrodes $C_i$ to another pair of electrodes $C_{i'}$ and to cover the distance separating said pairs $C_i$ and $C_{i'}$, said time period $\Delta T_{ii'}$ being equal to the time difference $T_{i'} - T_i$.

15. The method according to one of the claims 13 to 14, **characterized in that** the measured electric parameter $X_i$ is chosen from a conductivity, a voltage, a resistance, a capacitance and/or a global ion concentration.

Figure 1 (Art antérieur)

Figure 2 (Art antérieur)

Figure 3

Figure 4

Figure 5

Figure 6

Figure 7

Figure 8

Figure 9

Figure 10

Figure 11

Figure 12

Figure 13

Figure 14

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- JP 2010046196 A
- JP 9051877 A
- FR 2669529